# EUROPEAN PATENT SPECIFICATION

(11) **EP 2 615 457 B1**
(45) Date of publication and mention of the grant of the patent: **13.01.2016**
(21) Application number: 11823273.5
(22) Date of filing: 14.01.2011
(51) Int. Cl.: G01N 33/543, G01N 33/558, C12Q 1/68

(54) **REAGENT COMPOSITION FOR NUCLEIC ACID CHROMATOGRAPHY OR IMMUNOCHROMATOGRAPHY, METHOD FOR MEASUREMENT BY NUCLEIC ACID CHROMATOGRAPHY OR IMMUNOCHROMATOGRAPHY, AND KIT FOR MEASUREMENT BY NUCLEIC ACID CHROMATOGRAPHY OR IMMUNOCHROMATOGRAPHY**
REAGENZIENZUSAMMENSETZUNG ZUR NUKLEINSÄURE-CHROMATOGRAPHIE ODER IMMUNCHROMATOGRAPHIE, VERFAHREN FÜR MESSUNGEN DURCH NUKLEINSÄURE-CHROMATOGRAPHIE ODER IMMUNCHROMATOGRAPHIE UND KIT FÜR MESSUNGEN DURCH NUKLEINSÄURE-CHROMATOGRAPHIE ODER IMMUNCHROMATOGRAPHIE
COMPOSITION DE RÉACTIF POUR LA CHROMATOGRAPHIE OU L'IMMUNOCHROMATOGRAPHIE D'ACIDE NUCLÉIQUE, PROCÉDÉ DE MESURE PAR LA CHROMATOGRAPHIE OU L'IMMUNOCHROMATOGRAPHIE D'ACIDE NUCLÉIQUE, ET KIT DE MESURE PAR CHROMATOGRAPHIE OU IMMUNOCHROMATOGRAPHIE D'ACIDE NUCLÉIQUE

(30) Priority: 08.09.2010 JP 2010200793
(43) Date of publication of application: 17.07.2013
(73) Proprietor: Tanaka Kikinzoku Kogyo K.K., Chiyoda-ku Tokyo 100-6422 (JP)
(72) Inventor: SAKAKIBARA, Yuhiro, Kanagawa 254-0076 (JP)
(74) Representative: Hoffmann Eitle
(86) International application number: PCT/JP2011/050564
(87) International publication number: WO 2012/032794

(56) References cited:
- EP-A1- 2 108 961
- WO-A1-03/085402
- WO-A1-2010/007734
- WO-A2-02/085185
- JP-A- 10 332 699
- JP-A- 2002 122 598
- JP-A- 2007 248 073
- JP-A- 2008 164 334
- JP-A- 2009 250 763
- US-A- 4 931 385
- US-A1- 2008 108 147
- Product Specification 4-Aminoantipyrine: In: "Sigma Aldrich catalogue", 1 January 2014 (2014-01-01)
- "Hawley's Condensed Chemical Dictionary 14th ed.", 1 January 2001 (2001-01-01) page 642,

## Description

### TECHNICAL FIELD

The present invention relates to a reagent composition used for nucleic acid chromatography or immunochromatography, a method for measurement by nucleic acid chromatography or immunochromatography using the same, and a kit for measurement by nucleic acid chromatography or immunochromatography.

### BACKGROUND ART

In order to enhance the sensitivity in a measurement by nucleic acid chromatography or immunochromatography, there is conventionally an ordinary measure such as an increase of the amount of antibody or nucleic acid applied to a solid phase, or an increase of the amount of antibody or nucleic acid to be bound to a labeling substance.

However, an increase of the amount of reactant such as an antibody and a nucleic acid tends to cause a non-specific reaction, and a small amount of reactant causes a problem such as an insufficient sensitivity.

In nucleic acid chromatography or immunochromatography, various reagent components are used for a variety of purposes. Attempts have been made to add additives including a water-soluble polymer such as a polyanion, inorganic salts, and a surfactant to suppress a non-specific reaction or improve the sensitivity (Patent documents 1 to 11).

However, neither the suppression of a non-specific reaction, the improvement of the sensitivity, nor improvement of the developability on a chromatography carrier, has been fully achieved by any combination of the additives.

### PRIOR ART DOCUMENTS

### Patent documents

Patent document 1: Japanese Unexamined Patent Publication No. 2001-21560
Patent document 2: Japanese Unexamined Patent Publication No. 2006-215044
Patent document 3: Japanese Unexamined Patent Publication No. 2006-317226
Patent document 4: Japanese Unexamined Patent Publication No. 2007-121204
Patent document 5: Japanese Unexamined Patent Publication No. 2007-121205
Patent document 6: Japanese Unexamined Patent Publication No. 2007-322310
Patent document 7: Japanese Unexamined Patent Publication No. 2009-52945
Patent document 8: Japanese Unexamined Patent Publication No. 2010-14507
Patent document 9: Japanese Unexamined Patent Publication No. 2010-19794
Patent document 10: Japanese Unexamined Patent Publication No. 2010-44094
Patent document 11: Japanese Patent Publication No. 2010-50055

EP 2 108 961 A1, US 4 931 385 A, WO 02/085185 A2 and US 2008/108147 A1 mention reagent compositions for use in bioassays but do not disclose the specific reagent composition claimed herein. For example, EP 2 108 961 A1 does not disclose a reagent composition wherein the aprotic water-soluble organic compound is a ketone.

### DISCLOSURE OF INVENTION

### Problem to be solved by the invention

An object of the present invention is to determine an analyte accurately and rapidly even when it has a low concentration in the measurement by nucleic acid chromatography or immunochromatography, by reducing the binding of components other than the analyte through a non-specific reaction and enhancing the dispersion capability of the analyte to improve the developability on a chromatography carrier and promote a specific reaction.

### Means for solving problem

The present inventors have found that a combination of particular additives is used to solve the problems, and the present invention has been completed.

The present invention is a reagent composition for nucleic acid chromatography or immunochromatography comprising a water-soluble polymer having a weight average molecular weight of 8,000 or more, a salt of a divalent or trivalent metal, a nonionic surfactant, and an aprotic water-soluble organic compound which is selected from sulfoxides, N,N'-dialkylamides, ketones, nitriles, and cyclic ethers.

The present invention is a method for measurement by nucleic acid chromatography or immunochromatography including developing a sample to be measured using the reagent composition as defined in the claims for nucleic acid chromatography or immunochromatography.

The present invention is a kit for measurement by nucleic acid chromatography or immunochromatography containing the reagent composition as defined in the claims for chromatography as a developer for a sample to be measured.

### Effect of the invention

According to the present invention, an analyte can be accurately and rapidly determined even when it has a low concentration in the measurement by nucleic acid chromatography or immunochromatography, by reducing the binding of components other than the analyte through a non-specific reaction and enhancing the dispersion capability of the analyte to promote a specific reaction.

### BRIEF DESCRIPTION OF THE DRAWINGS

Fig. 1 is a schematic process view of measurement by nucleic acid chromatography.
Fig. 2 is a schematic view of a kit for measurement by nucleic acid chromato graphy.
Fig. 3 is a schematic view illustrating the principle of measurement by nucleic acid chromatography.

### BEST MODE FOR CARRYING OUT THE INVENTION

The reagent composition for nucleic acid chromatography or immunochromatography of the present invention comprises a water-soluble polymer having a weight average molecular weight of 8,000 or more, a salt of a divalent or trivalent metal, a nonionic surfactant, and an aprotic water-soluble organic compound which is selected from sulfoxides, N,N'-dialkylamides, ketones, nitriles, and cyclic ethers.

### (Water-soluble polymer having a weight average molecular weight of 8,000 or more)

The water-soluble polymer having a weight average molecular weight of 8,000 or more is not particularly limited as long as it is a compound having a weight average molecular weight of 8,000 or more and water solubility. It is preferable that the weight average molecular weight be 10,000 or more. As the water-soluble polymer, a commercially available compound in which the weight average molecular weight is known can be used as long as the weight average molecular weight is 8,000 or more. When a synthesized compound is used, the weight average molecular weight thereof determined by gel permeation chromatography (hereinafter referred to as GPC) which is generally used in measurement of molecular weight of polymer is 8,000 or more. When the weight average molecular weight is less than 8,000, the strength of a binding forming a specific complex with an analyte in hybridization of nucleic acid or an antigen-antibody reaction is reduced, and therefore the detection sensitivity by chromatography is reduced. A water-soluble polymer having a weight average molecular weight of 20,000 to 1,000,000 is more preferably used. The term "water solubility" of the water-soluble polymer having a weight average molecular weight of 8,000 or more is that the compound in an amount of 10g or more, and preferably 50 g or more, is dissolved in 1 L of water at 20°C.

The water-soluble polymer having a weight average molecular weight of 8,000 or more is for example, one or more kinds selected from the group consisting of polyalkylene glycols such as polyethylene glycol, polypropylene glycol, and a polyethylene glycol-polypropylene glycol block copolymer; celluloses such as methyl cellulose, ethyl cellulose, hydroxyethyl cellulose, hydroxypropyl cellulose, hydroxypropylmethyl cellulose, and carboxymethyl cellulose; vinyl-based polymers such as polyvinyl pyrrolidone, polyvinyl alcohol, and polyvinyl methyl ether; amide-based polymers such as polymethacrylamide and polyacrylamide; and a polyanion.

Examples of a polyanion include a polysaccharide anion, a synthetic peptide-based anion such as polyglutamic acid and polyaspartic acid, and a synthetic nucleic acid-based polyanion. A polysaccharide anion is preferable.

As a polysaccharide anion, one or more kinds are selected from the group consisting of dextran sulfate, heparan sulfate, chondroitin sulfate, dermatan sulfate, keratan sulfate, hyaluronic acid, heparin, and salts thereof, for example. The salts include sodium, potassium, and magnesium salts. In particular, dextran sulfate and a salt thereof are more preferable in terms of ease of acquisition and economy.

The amount of water-soluble polymer having a weight average molecular weight of 8,000 or more is preferably 0.1 to 5% by weight, and more preferably 0.5 to 3% by weight, relative to the reagent composition.

The water-soluble polymer having a weight average molecular weight of 8,000 or more enhances the strength of a binding forming a specific complex with an analyte and promotes a specific reaction.

### (Salt of divalent or trivalent metal)

Examples of a divalent or trivalent metal include, but not particularly limited to, an alkaline earth metal (divalent) such as magnesium and calcium, and an aluminum group metal (trivalent) such as boron and aluminum. Magnesium, calcium, or aluminum is preferable, magnesium or calcium is particularly preferable, and magnesium is most preferable. Use of an alkali metal (monovalent) may decrease a development speed and increase a non-specific reaction.

Examples of an anion of a divalent or trivalent metal salt include, but not particularly limited to, a halide such as a chloride and a bromide; a sulfate; a phosphate; a carbonate; and a borate. A halide such as a chloride and a bromide is preferably used.

The salt of a divalent or trivalent metal is preferably one or more kinds selected from the group consisting of a magnesium salt such as magnesium chloride, magnesium sulfate, magnesium phosphate, and magnesium carbonate; a calcium salt such as calcium chloride, calcium sulfate, and calcium phosphate; and an aluminum salt such as aluminum chloride, aluminum sulfate, and aluminum carbonate.

The amount of salt of a divalent or trivalent metal is preferably 0.1 to 100 mM, more preferably 0.5 to 50 mM, and further preferably 1 to 10 mM, relative to the reagent composition.

The salt of a divalent or trivalent metal does not cause a non-specific reaction of immune complex and increases the development speed of capillary phenomenon.

### (Nonionic surfactant)

Examples of a nonionic surfactant include polyoxyethylene alkyl ether, polyoxyethylene alkyl phenol ether, alkylglucoside, a polyoxyethylene fatty acid ester, a sucrose fatty acid ester, a sorbitan fatty acid ester, a polyoxyethylene sorbitan fatty acid ester, and a fatty acid alkanolamide. The HLB of nonionic surfactant is preferably 10 to 18, and more preferably 13 to 18. The nonionic surfactant is preferably a polyoxyethylene sorbitan fatty acid ester, and particularly preferably one or more kinds selected from the group consisting of polyoxyethylene sorbitan monolaurate, polyoxyethylene sorbitan monostearate, polyoxyethylene sorbitan tristearate, and polyoxyethylene sorbitan monooleate, for example.

The amount of a nonionic surfactant is preferably 0.1 to 5% by weight, more preferably 0.5 to 2.5% by weight, and further preferably 0.5 to 1% by weight, relative to the reagent composition.

### (Aprotic water-soluble organic compound)

An aprotic water-soluble organic compound disclosed herein is not particularly limited as long as it is an organic compound which is water soluble but not protic. The term "water soluble of an organic compound means a property that the organic compound can be mixed with water in any ratio without phase separation. The preferable aprotic water-soluble organic compound is an organic compound in which the amount dissolved in 1 L of water at 20°C is 10 g or more, and preferably 50 g or more. The term "aprotic" of an organic compound means a property that the organic compound does not contain an acidic hydrogen and act as a hydrogen bond donor.

The water-soluble organic compound used in the present invention are sulfoxides, N,N'-dialkylamides, ketones, nitriles, and cyclic ethers.

Examples of sulfoxides include dimethyl sulfoxide, methyl ethyl sulfoxide, diethyl sulfoxide, and butyl ethyl sulfoxide.

Examples of N,N'-dialkylamides include dialkylacetamide such as dimethylacetamide, dialkylformamide such as dimethylformamide, N-alkylpyrrolidone such as N-methyl-pyrrolidone, N-ethyl-pyrrolidone, and N-(2-hydroxyethyl)-2-pyrrolidone.

Examples of ketones include acetone, acetyl acetone, diethyl ketone, methyl ethyl ketone, methyl propyl ketone, isobutyl methyl ketone, γ-butyrolactone, and γ-valerolactone.

Examples of nitriles include acetonitrile, propionitrile, and butyronitrile.

Examples of cyclic ethers include tetrahydrofuran, 2-methyltetrahydrofuran, and 1,4-dioxane.

The aprotic water-soluble organic compound is preferably sulfoxides or N,N'-dialkylamides, and particularly preferably sulfoxides.

The amount of aprotic water-soluble organic compound is preferably 0.2 to 5% by weight, and more preferably 0.5 to 3% by weight, relative to the reagent composition.

The aprotic water-soluble organic compound improves non-uniformity of a developer caused by fusion and aggregation of substances other than analytes in development due to a capillary phenomenon, and therefore is unlikely to cause a non-specific reaction.

The reagent composition of the present invention usually contains water as a solvent. The components described above are mixed in water, for example, e-pure water to obtain the reagent composition.

### (Nucleic acid chromatography or immunochromatography)

The reagent composition of the present invention is used in nucleic acid chromatography or immunochromatography.

Nucleic acid chromatography is based on hybridization of a nucleic acid.

Immunochromatography is not particularly limited as long as it is based on an antigen-antibody reaction, and examples thereof include a competitive method and a sandwich method. In particular, the sandwich method is generally used.

In nucleic acid chromatography or immunochromatography, colloidal particles of noble metal such as gold, silver, or platinum, colloidal particles of metal oxide such as iron oxide, and latex particles can be used as a labeling substance to label a nucleic acid, an antigen, and the like, which are analytes. Colloidal gold particles are preferably used. The mean particle size of the colloidal metal particles falls within a range of 1 to 500 nm, preferably 10 to 150 nm, and more preferably 40 to 100 nm since particularly strong tone is obtained. The labeling substance is complexed with a protein having a binding capacity to an analyte, or with a complementary nucleic acid or antibody having a specific binding capacity, and the complex is used as a labeling reagent for labeling the analyte. When the labeling reagent is developed on a chromatography medium, the labeling reagent may be developed simultaneously with a sample containing the analyte or after the development. The labeling reagent is dried and held at an arbitrary area on the chromatograph medium, a developer or a diluted solution of the sample is supplied and applied to an upper sample pad (sample application area) for development. Thus, the labeling reagent is dissolved from the labeling reagent-holding area for development. Further, the labeling reagent can be used to prepare a dispersion solution by dispersed in the developer or diluted solution, and the dispersion solution is developed on the chromatography medium. It is preferable that the labeling reagent be dried and held at an arbitrary area on the chromatography medium in advance in terms of preservation.

It is preferable that the reagent composition of the present invention contain a blocking agent, for example, protein such as bovine serum albumin, protein derived from milk, skim milk, casein, and gelatin, and a commercially available hydrophilic macromolecular polymer such as Blocking Peptide Fragment (TOYOBO), modified-fish-DNA, tRNA derived from yeast, and CE510 (JSR Corporation). When the reagent composition contains a blocking agent, the background can be reduced without reduction of sensitivity, and therefore an S/N ratio can be improved.

The reagent composition of the present invention can contain phosphate, tris(hydroxymethyl)aminomethane hydrochloride, carbonate, an amino acid such as glycine, a buffer such as a Good buffer, and components conventionally used as a reagent composition for nucleic acid chromatography or immunochromatography as long as effects of the present invention are exhibited.

The reagent composition of the present invention can be used as a developer in nucleic acid chromatography or immunochromatography. Water is typically used as a solvent of a developer. A water-soluble polymer having a weight average molecular weight of 8,000 or more, a salt of a divalent or trivalent metal, a nonionic surfactant, and an aprotic water-soluble organic compound are added to the solvent. The addition order is not particularly limited, and they may be added at once. When the reagent composition is used as a developer, a sample containing an analyte to be detected and a developer are mixed in advance, and the mixture may be supplied and applied to a chromatography medium, a labeling reagent-holding area, or a sample pad for development. Alternatively, the sample may be supplied and applied to the sample pad in advance, and then the developer may be supplied and applied to the sample pad for development. When the reagent composition is used directly as a diluted solution of a sample, the diluted solution of a sample can be used as a developer by being supplied and applied to a chromatography medium, a labeling reagent-holding area, or a sample pad. In addition, the reagent composition of the present invention can be held in advance in a sample pad or a drying and holding area of a labeling reagent in nucleic acid chromatography or immunochromatography. The reagent composition of the present invention which is used as a diluent or held in a sample pad or a drying and holding area of a labeling reagent is used as a developer or a part thereof in the following steps. When the reagent composition is held in a drying and holding area of a labeling reagent, some aggregation may occur during development. Therefore, it is preferable that the reagent composition be held in advance in a developer, a diluted solution, or a part thereof, or in a sample pad.

The analyte of the present invention is not particularly limited as long as a substance specifically bound to it exists or is producible. The term "specifically binding" means that binding is based on the affinity of a biomolecule. A representative of such a binding based on such an affinity is a binding between an antigen and an antibody is, and is widely used in an immunoassay. In addition to such a binding, a binding between of a saccharide and a lectin, a binding between of a hormone and a receptor, a binding between of an enzyme and an inhibitor, a binding between of a nucleic acid and a complementary nucleic acid, or a binding between of a nucleic acid and a protein having a binding capacity to the nucleic acid can be used in the present invention. The analyte may be a complete antigen which has antigenicity by itself or a hapten (incomplete antigen) which does not have antigenicity by itself but has antigenicity when it is converted into a chemically modified compound. A detection substance specifically bound to the analytes may exist or be producible. Examples of the detection substance include a nucleic acid or a nucleic acid-binding protein complementary to the nucleic acid of the analyte, a monoclonal antibody, and a polyclonal antibody. Examples of the analyte of the present invention include a nucleic acid (single-stranded nucleic acid or double-stranded nucleic acid) in a cultured cell strain, peripheral blood, or microorganisms such as bacteria and virus, or an amplified substance thereof, carcinoembryonic antigen (CEA), HER2 protein, prostate-specific antigen (PSA), CA19-9, α-fetoprotein (AFP), immunosuppressive acidic protein (IPA), CA15-3, CA125, an estrogen receptor, a progesterone receptor, fecal occult blood, troponin I, troponin T, CK-MB, CRP, human chorionic gonadotropin (HCG), luteinizing hormone (LH), follicle stimulating hormone (FSH), a syphilis antigen, influenza virus, a chlamydiae antigen, a group A β-streptococcal antigen, a HBs antibody, a HBs antigen, rotavirus, adenovirus, albumin, glycated albumin, allergens of pollen, tick, house dust, foods, and the like, and allergen specific IgE. A nucleic acid in a cultured cell strain, peripheral blood, or microorganisms such as bacteria and virus is preferably used. Specific examples thereof include DNA, RNA, oligonucleotide, polynucleotide, and an amplified substance thereof. In this case, the analyte may be a nucleic acid itself or a nucleic acid modified by a ligand or a binding protein having a binding capacity to a labeling reagent.

Examples of a sample containing a substance to be detected include a biological sample, that is, whole blood, serum, plasma, urine, saliva, expectoration, nasal or throat swab, spinal fluid, amniotic fluid, nipple discharge, tear, sweat, a percolate from the skin, and extracts from tissues, cells, and feces, and extracts from milk, egg, wheat, bean, beef, pork, chicken, and food containing them. Further, examples thereof include, but not limited to, an extract of nucleic acid in a cultured cell strain, peripheral blood, or microorganisms such as bacteria and virus, a fluid containing an amplified substance of the extracted nucleic acid, and a fluid containing a nucleic acid modified by a ligand or a binding protein having a binding capacity to the nucleic acid.

Hereinafter, a detection method, a determining method, and the principle of detection in nucleic acid chromatography will be described with reference to a sandwich method.

### (Example of detection method)

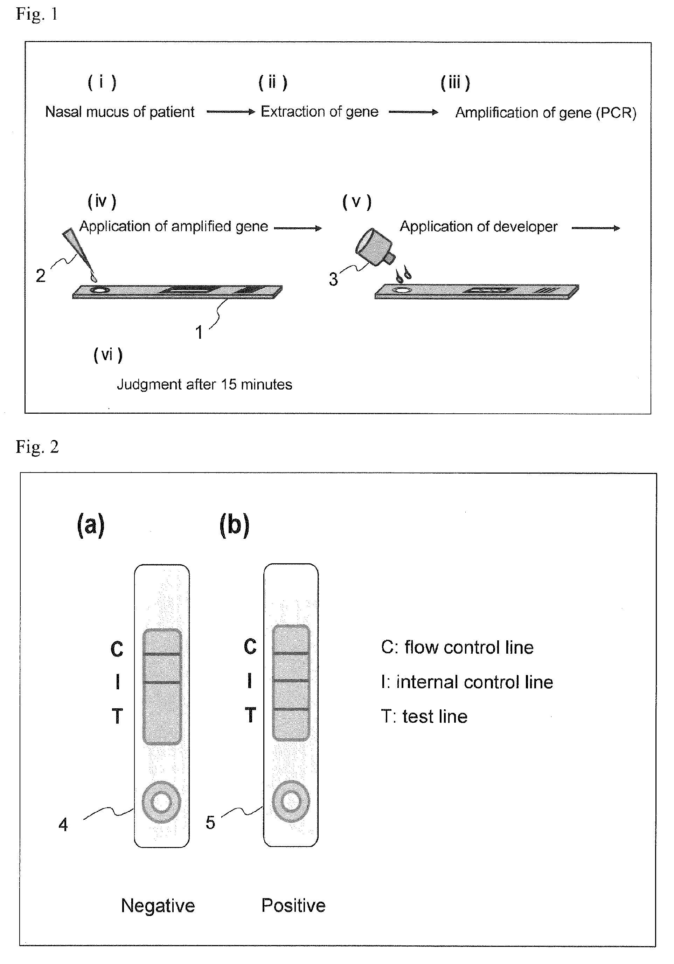

Fig. 1 shows an example of a detection method. (i) An analysis sample (e.g., nasal mucus) is collected. (ii) Genes of virus and the like are extracted from the analysis sample. (iii) If necessary, the extracted genes and the like are amplified with a gene amplification device by the PCR method. (iv) The genes and the like are added to a kit for measurement of immunity. (v) A developer is applied to develop the genes and the like. (vi) For example, after 15 minutes, the sample is determined to be positive or negative.

### (Determining method)

Fig. 2 shows a determining method. A sample is determined to be positive or negative by the presence or absence of a red line. When a red line appears on a Test line (T position), a target virus and the like is positive (Fig. 2(a)), and when it does not appear, the target virus and the like is negative (Fig. 2(b)). At this time, an internal control line (I position) indicates detection of a normal human cell of a patient contained when a specimen such as viruses is collected from the nose of the patient. Therefore, when an analysis sample is not properly collected during collection of specimen (e.g., when the nasal cavity is not sufficiently swabbed), or when a problem is caused in amplification of gene (PCR), the internal control line does not appear. The test is to be performed again. When a Flow control line (C position) does not appear, a problem is caused in measurement. As a result, the test is to be performed again.

### (Detection principle)

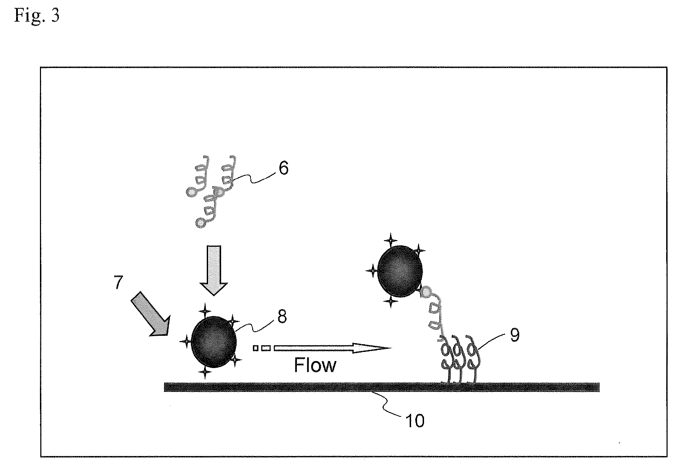

Fig. 3 shows detection principle. An amplified gene (e.g., single-stranded DNA modified by biotin and amplified) which is added to a kit for measurement of immunity, and the like, are bound to a labeling substance (e.g., colloidal gold), for example, via biotin-streptavidin. After then, when a developer is applied, an amplified gene-colloidal gold complex migrates on DNA immobilized in T and I lines by a capillary phenomenon. In advance, a complementary DNA which is bound to only DNA derived from a target virus is immobilized in the T line, and a complementary DNA which is bound to only DNA derived from a normal human cell is immobilized in the I line. Therefore, when the result is positive, a red line based on colloidal gold is formed.

For example, a biotin-labeling protein to be bound to a labeling substance (e.g., streptavidin-binding colloidal gold) is immobilized in the C line. When the development is good, a red line based on colloidal gold appears on the C line.

The present invention is a method for measurement by nucleic acid chromatography or immunochromatography, which includes developing a sample to be measured using the reagent composition as defined in the claims. This step includes an aspect of using the reagent composition as a diluent for a sample to be measured, or previously holding the reagent composition in a sample pad and then using it as a part of a developer in the following development.

The present invention is a kit for measurement by nucleic acid chromatography or immunochromatography, which includes the reagent composition as defined in the claims as a developer for a sample to be measured. This kit includes an aspect of containing the reagent composition as a diluent for a sample to be measured, or previously holding the reagent composition in a sample pad and then using it as a diluent after dilution. Therefore, the developer can be used to be mixed in a sample containing an analyte to be detected in advance, and the mixture can be supplied and applied to a chromatography medium, a labeling reagent-holding area, or a sample pad, for development. Alternatively, the sample may be supplied and applied to a sample pad in advance, and then the developer may be supplied and applied to the sample pad for development. When the developer is used as a diluted solution of a sample, the diluted solution of a sample can be used as a developer by being supplied and applied to a chromatography medium, a labeling reagent-holding area, or a sample pad. In addition, the developer can be held in advance in a sample pad or a drying and holding area of a labeling reagent in nucleic acid chromatography or immunochromatography. The developer which is used as a diluent or held in a sample pad or a drying and holding area of a labeling reagent is used as a whole or a part of the developer in the following development step. When the developer is held in a drying and holding area of a labeling reagent, some aggregation may occur during development, and therefore it is not preferable. Accordingly, it is preferable that the reagent composition be held in a developer (including use as a diluted solution) or a part thereof, or in a sample pad, in advance.

### EXAMPLES

The present invention will be described in more detail by Examples and Comparative Examples. Percentages are based on weight.

### 1. Nucleic acid chromatography

### [1. Preparation of diluted solution for capture probe]

87.7 g of sodium chloride and 44.1 g of sodium citrate dihydrate were weighed, and dissolved in 800 mL of purified water to adjust the pH to 7.0. The solution was filled up to 1,000 mL, and sterilized in an autoclave to prepare a diluted solution for a capture probe.

### [2. Production of reaction portion on chromatography medium]

An influenza A virus capture probe was diluted with the diluted solution for a probe to a concentration of 2.0 µM. This solution was applied to a 25 x 2.5 cm nitrocellulose membrane (manufactured by Millipore) with a coating machine (manufactured by BioDot), followed by drying at 50°C for 5 minutes and then at 80°C for 1 hour to produce a reaction portion on a chromatography medium.

### [3. Preparation of labeling reagent solution]

1 mL of 40 mM phosphate buffer solution (pH: 8.5) was added to 20 mL of colloidal gold suspension (manufactured by Tanaka Kikinzoku Kogyo K.K., average particle diameter: 40 nm) and the mixture was stirred. 4 mL of streptavidin diluted with a phosphate buffer solution (pH: 8.5) to a concentration of 0.01 mg/mL was added, and the mixture was left to stand at room temperature for 15 minutes. 1 mL of CE510 (available from JSR Corporation) was then added and the mixture was left to stand at room temperature for 15 minutes. The mixture was centrifuged at 8000 x g for 15 minutes. After the centrifugation, the supernatant was removed, and 4 mL of buffer (pH: 7.4) containing 1% by weight bovine serum albumin (BSA) was added to the residue to prepare a labeling reagent solution.

### [4. Production of chromatography medium]

400 µL of the labeling reagent solution prepared in 3 was uniformly applied to a glass fiber pad (16 mm x 100 mm), and the pad was dried with a vacuum dryer to obtain a labeling reagent-holding member. Then, the chromatography medium, the labeling reagent-holding member, a sample pad (manufactured by Millipore, 300 mm x 30 mm) to be used as a portion to which a sample is applied, and an absorbing pad for absorbing the developed sample and an excessive labeling reagent were bonded to a base material of a backing sheet. The obtained sheet was cut to a width of 5 mm with a cutter to obtain a chromatography medium.

### [5. Preparation of developer]

### (Examples 1 to 8 and Comparative Examples 1 to 4)

10% Tween20, 0.1 M magnesium sulfate, dimethyl sulfoxide, and 20% dextran sulfate sodium (weight average molecular weight: 500,000), in amounts shown in Table 1, and CE510 (available from JSR Corporation) in such an amount that the concentration was 2% were mixed in e-pure water. In addition, 10% sodium azide in such an amount that the concentration was 0.05% was added as a preservative and mixed to obtain a reagent composition.

**Table 1**

| Example / Comparative example | Dextran sulfate sodium (%) | Magnesium sulfate (mM) | Dimethyl sulfoxide (%) | Tween20 (%) |
|---|---|---|---|---|
| Comparative example 1 | 0 | 5 | 0.95 | 1 |
| Example 1 | 0.5 | 5 | 0.95 | 1 |
| Example 2 | 3 | 5 | 0.95 | 1 |
| Comparative example 2 | 2 | 0 | 0.95 | 1 |
| Example 3 | 2 | 1 | 0.95 | 1 |
| Example 4 | 2 | 20 | 0.95 | 1 |
| Comparative example 3 | 2 | 5 | 0 | 1 |
| Example 5 | 2 | 5 | 0.5 | 1 |
| Example 6 | 2 | 5 | 3 | 1 |
| Comparative example 4 | 2 | 5 | 0.95 | 0 |
| Example 7 | 2 | 5 | 0.95 | 0.5 |
| Example 8 | 2 | 5 | 0.95 | 2.5 |

### (Examples 9 to 14 and Comparative Examples 5 to 9)

Components of types shown in Table 2 in such amounts that the concentrations of a water-soluble polymer having a weight average molecular weight of 8,000 or more, a salt of divalent or a trivalent metal, a nonionic surfactant, an aprotic water-soluble organic compound, and CE510 (available from JSR Corporation) were 2%, 5 mM, 1%, 0.95%, and 2%, respectively, were mixed in e-pure water. In addition, 10% sodium azide in such an amount that the concentration was 0.05% was added as a preservative and mixed to obtain reagent compositions of Examples 9 to 14.

Components of types shown in Table 2 in such amounts that the concentrations of a water-soluble polymer, a metal salt, a nonionic surfactant, isopropanol CE510, and sodium azide were 2%, 5 mM, 1%, 0.95%, 2%, and 0.05% ,respectively, were mixed in e-pure water, to obtain reagent compositions of Comparative Examples 5 to 9.

**Table 2**

| Component | | Example | | | | | | Comparative Example | | | | |
|---|---|---|---|---|---|---|---|---|---|---|---|---|
| | | 9 | 10 | 11 | 12 | 13 | 14 | 5 | 6 | 7 | 8 | 9 |
| Water-soluble polymer | Dextran sulfate sodium (Weight average molecular weight: 500,000) | ○ | | | ○ | ○ | ○ | | | ○ | ○ | ○ |
| | Sodium hyaluronate (Weight average molecular weight: 150,000) | | ○ | | | | | | | | | |
| | Polyethylene glycol (Weight average molecular weight: 20,000) | | | ○ | | | | | | | | |
| | Polyethylene glycol (Weight average molecular weight: 6,000) | | | | | | | ○ | | | | |
| | Dextran (Weight average molecular weight: 6,000) | | | | | | | | ○ | | | |
| Salt of divalent or trivalent metal | Magnesium sulfate | ○ | ○ | ○ | | | ○ | ○ | ○ | | ○ | ○ |
| | Calcium chloride | | | | ○ | | | | | | | |
| | Aluminum carbonate | | | | | ○ | | | | | | |
| | Lithium chloride, sodium chloride, potassium chloride, or sodium phosphate | | | | | | | | | ○ | | |
| Aprotic water-soluble organic compound | DMSO | ○ | ○ | ○ | ○ | ○ | | ○ | ○ | ○ | ○ | ○ |
| | NMP | | | | | | ○ | | | | | |
| | Isopropanol | | | | | | | | | | ○ | |
| Nonionic surfactant | Polyoxyethylene sorbitan monolaurate | ○ | ○ | ○ | ○ | ○ | | ○ | ○ | ○ | ○ | |
| | Polyoxyethylene sorbitan monostearate | | | | | | ○ | | | | | |
| | Sodium cholate (anionic surfactant) | | | | | | | | | | | ○ |

### [6. Measurement]

The presence or absence of influenza A virus in a sample was determined using the chromatography medium as prepared above according to the following process. In amplification of gene of influenza A virus extracted from nasal mucus of a patient infected with influenza virus, the gene was modified with biotin and amplified to a single-stranded nucleic acid. Thus, 2.0 x10⁶ copies/µL (copy: one molecule of copy) of positive specimen was obtained. 15 µL of the specimen was applied to a sample application window. Then, 100 µL of developer was applied to the sample application window immediately. After 15 minutes, the presence or absence of a line was observed. Further, a negative specimen collected from a normal subject which was not infected with influenza A virus was also extracted from nasal mucus, produced, and measured in the same manner.

### [7. Test Example 1]

A relationship between an analyte (DNA amount) and color strength of a line (i.e., coloring strength) is considered to be y=βM (Table 4). This is because since the amount of complementary DNA immobilized in colloidal gold and a nitrocellulose membrane is abundant, the amount of complex formed on a T or I line of the nitrocellulose membrane is increased according to the amount of DNA to be added to obtain a strong coloring strength. When the analysis sample does not contain DNA, only colloidal gold is developed in conjunction with a developer, but the colloidal gold does not react with DNA immobilized in the membrane, and a T line is not formed.

Coloring of 4-fold, 6-fold, and 8-fold diluted solutions of positive specimen (2.0 x 10⁶ copies/µL) and negative specimen and unevenness of development were tested using the reagent compositions of Examples 1 to 8 and Comparative Examples 1 to 4 as a developer.

In coloring, coloring strength was evaluated visually in accordance with the following criteria 10 minutes after development of a sample.
+++ : a sample that showed a very strong red line of labeling substance
++ : a sample that showed a strong red line of labeling substance
+ : a sample that showed a red line of labeling substance
± : a sample that was weakly colored and unlikely to show a red line of labeling substance
- : a sample that did not show a red line of labeling substance

The unevenness of development was evaluated through a method for observing a flowing form of edge of red colloidal gold in the development.

The test results are shown in Table 3.

**Table 3**

| Example / Comparative example | Negative specimen | Positive specimen | | | Unevenness of development |
|---|---|---|---|---|---|
| | | 8-Fold dilution | 6-Fold dilution | 4-Fold dilution | |
| Comparative example 1 | - | - | - | - | None |
| Example 1 | - | + | + | ++ | None |
| Example 2 | - | + | ++ | +++ | None |
| Comparative example 2 | + | + | ++ | +++ | Presence |
| Example 3 | - | + | ++ | +++ | None |
| Example 4 | - | + | + | ++ | None |
| Comparative example 3 | + | + | ++ | +++ | Presence |
| Example 5 | - | + | ++ | +++ | None |
| Example 6 | - | + | ++ | +++ | None |
| Comparative example 4 | - | - | ± | ± | None |
| Comparative example 7 | - | + | ++ | +++ | None |
| Comparative example 8 | - | + | ++ | +++ | None |

### [8. Test Example 2]

Coloring of 4-fold, 6-fold, and 8-fold diluted solutions of positive specimen and negative specimen and unevenness of development were tested using the reagent compositions of Examples 9 to 14 and Comparative Examples 5 to 9 as a developer. The results are shown in Table 4.

**Table 4**

| Coloring strength | | Example | | | | | | Comparative example | | | | |
|---|---|---|---|---|---|---|---|---|---|---|---|---|
| | | 9 | 10 | 11 | 12 | 13 | 14 | 5 | 6 | 7 | 8 | 9 |
| Negative specimen | | - | - | - | - | - | - | - | - | + | ± | - |
| Positive specimen | 8-Fold dilution | + | + | ± | + | + | + | - | - | + | + | - |
| | 6-Fold dilution | ++ | ++ | + | ++ | + | ++ | - | - | ++ | ++ | ± |
| | 4-Fold dilution | +++ | ++ | ++ | ++ | ++ | ++ | - | ± | +++ | +++ | ± |
| Unevenness of development | | None | None | None | None | None | None | Presence | None | Presence | Presence | None |

### 2. Immunochromatography

### [1. Preparation of diluted solution for capture antibody]

Isopropyl alcohol in such an amount that the concentration was 5% was mixed and dissolved in 50 mM phosphate buffer solution (pH: 7.4) to prepare a diluted solution for a capture antibody.

### [2. Production of reaction portion on chromatography medium]

Influenza A virus capture antibody (mouse-derived anti-influenza A monoclonal antibody (first antibody)) was diluted with the diluted solution for an antibody to a concentration of 1.0 mg/mL. This solution was applied to a 25 x 2.5 cm nitrocellulose membrane (manufactured by Millipore) with a coating machine (manufactured by BioDot), followed by drying at 50°C for 5 minutes and then at room temperature for 1 hour to produce a reaction portion on a chromatography medium.

### [3. Preparation of labeling material solution]

0.1 mL of mouse-derived anti-influenza A monoclonal antibody (second antibody) diluted with a phosphate buffer solution (pH: 7.4) to a concentration of 0.05 mg/mL was added to 0.5 mL of colloidal gold suspension (manufactured by Tanaka Kikinzoku Kogyo K.K., average particle diameter: 40 nm), and the resulting mixture was left to stand at room temperature for 10 minutes. Then, 0.1 mL of phosphate buffer solution (pH: 7.4) containing 1% by weight bovine serum albumin was added and the mixture was left to stand at room temperature for 10 minutes. The mixture was sufficiently stirred and then centrifuged at 8000 x g for 15 minutes. The supernatant was removed, and 2 mL of phosphate buffer solution (pH: 7.4) containing 0.5% by weight BSA was added to the residue. By the procedure described above, a labeling reagent solution was prepared.

### [4. Production of chromatography medium]

The labeling reagent solution as described above was uniformly applied to a 15 mm x 300 mm glass fiber pad (manufactured by Millipore), and the pad was dried with a vacuum dryer to produce a labeling reagent-holding member. Then, the chromatography medium, the labeling reagent-holding member, a sample pad (manufactured by Millipore) to be used as a portion to which a sample was applied, and an absorbing pad for absorbing the developed sample and an excessive labeling reagent were bonded to a base material of a backing sheet. The obtained sheet was cut to a width of 5 mm with a cutter to obtain a chromatography medium.

### [5. Preparation of developer]

### (Example 15 and Comparative Examples 10 to 13)

10% Tween20, 0.1 M magnesium sulfate, dimethyl sulfoxide, 20% dextran sulfate sodium (weight average molecular weight: 500,000), and CE510 (available from JSR Corporation) in such amount that the concentrations were 1%, 5 mM, 0.95%, 2%, and 2%, respectively, were mixed in e-pure water as shown in Table 5. In addition, 10% sodium azide in such an amount that the concentration was 0.05% was added as a preservative and mixed to obtain reagent compositions of Example 15 and Comparative Examples 10 to 13.

**Table 5**

| Component | Addition amount | Example 15 | Comparative example 10 | Comparative example 11 | Comparative example 12 | Comparative example 13 |
|---|---|---|---|---|---|---|
| Dextran sulfate sodium | 2 % | ○ | | ○ | ○ | ○ |
| Magnesium sulfate | 5 mM | ○ | ○ | | ○ | ○ |
| DMSO | 0.95 % | ○ | ○ | ○ | | ○ |
| Tween 20 | 1 % | ○ | ○ | ○ | ○ | |

### [6. Measurement]

The presence or absence of influenza A virus in a sample was determined using the chromatography medium as prepared above according to the following process. A tube attached to a suction trap was connected with a suction pump, and another tube was deeply inserted into the nasal cavity of a person which was not infected with influenza A. Then, negative pressure was applied to the suction pump to collect nasal mucus. The collected nasal mucus was diluted 20-fold with the prepared developer to prepare a negative specimen. Further, a commercially available inactivated influenza A virus was added in such amounts that the protein concentration was 25 ng/mL and 50 ng/mL to the negative specimen to prepare positive specimens.

### [7. Test Example 1]

150 µL of each of negative specimen and positive specimens was applied to a sample pad including a specimen for immunochromatography and developed. After 15 minutes, the results were visually judged. The standards of coloring strength and unevenness of development were the same as in the nucleic acid chromatography.

The test results are shown in Table 6.

**Table 6**

| Coloring strength | | Example 15 | Comparative example 10 | Comparative example 11 | Comparative example 12 | Comparative example 13 |
|---|---|---|---|---|---|---|
| Negative sample | | - | - | ± | ± | - |
| Positive sample | 8-Fold dilution | + | ± | + | + | ± |
| | 6-Fold dilution | ++ | + | ++ | ++ | + |
| | 4-Fold dilution | ++ | + | ++ | ++ | ++ |
| Unevenness of development | | None | None | None | Presence | None |

### Industrial Applicability

According to the present invention, an analyte can be accurately and rapidly determined even when it has a low concentration in the measurement by nucleic acid chromatography or immunochromatography, by reducing the binding of components other than the analyte through a non-specific reaction and enhancing the dispersion capability of the analyte to promote a specific reaction.

### DESCRIPTION OF REFERENCE NUMERAL

1: Chromatography medium
2: Amplified gene
3: Developer
4: Negative coloring strip
5: Positive coloring strip
6: Amplified DNA
7: Developer
8: Colloidal gold
9: Membrane-immobilized probe
10: Nitrocellulose membrane

## Claims

1. A reagent composition for nucleic acid chromatography or immunochromatography comprising:
a water-soluble polymer having a weight average molecular weight of 8,000 or more,
a salt of a divalent or trivalent metal,
a nonionic surfactant,
and an aprotic water-soluble organic compound which is selected from sulfoxides, N,N'-dialkylamides, ketones, nitriles, and cyclic ethers.

2. The reagent composition according to claim 1, wherein the water-soluble polymer having a weight average molecular weight of 8,000 or more is one or more kinds selected from the group consisting of polyalkylene glycols, celluloses, vinyl-based polymers, amide-based polymers, and a polyanion.

3. The reagent composition according to claim 2, wherein the polyanion is a polysaccharide anion.

4. The reagent composition according to claim 3, wherein the polysaccharide anion is one or more kinds selected from the group consisting of dextran sulfate, heparan sulfate, chondroitin sulfate, dermatan sulfate, keratan sulfate, hyaluronic acid, heparin, and salts thereof.

5. The reagent composition according to any one of claims 1 to 4, wherein the aprotic water-soluble organic compound is one or more kinds selected from the group consisting of sulfoxides and N,N'-dialkylamides.

6. The reagent composition according to any one of claims 1 to 5, wherein the salt of a divalent or trivalent metal is one or more kinds selected from the group consisting of a magnesium salt, a calcium salt, and an aluminum salt.

7. The reagent composition according to any one of claims 1 to 6, wherein the nonionic surfactant is one or more kinds selected from the group consisting of polyoxyethylene sorbitan monolaurate, polyoxyethylene sorbitan monostearate, polyoxyethylene sorbitan tristearate, and polyoxyethylene sorbitan monooleate.

8. Use of the reagent composition according to any one of claims 1 to 7 for in nucleic acid chromatography or immunochromatography, wherein colloidal gold particles are used as a labeling substance.

9. Use of the reagent composition according to any one of claims 1 to 7 for nucleic acid chromatography.

10. Use of the reagent composition according to any one of claims 1 to 7 for immunochromatography.

11. Use of the reagent composition according to any one of claims 1 to 7 as a developer in nucleic acid chromatography or immunochromatography.

12. A method for measurement by nucleic acid chromatography or immunochromatography comprising developing a specimen to be measured using the reagent composition according to any one of claims 1 to 7.

13. A kit for measurement by nucleic acid chromatography or immunochromatography comprising the reagent composition according to any one of claims 1 to 7 as a developer of a specimen to be measured.

## Patentansprüche

1. Reagenz-Zusammensetzung für die Nukleinsäure-Chromatographie oder Immunochromatographie, umfassend:
ein wasserlösliches Polymer mit einem Gewichtsmittel der Molmasse von 8.000 oder mehr,
ein Salz eines zweiwertigen oder dreiwertigen Metalls, ein nicht-ionisches Tensid,
und eine aprotische, wasserlösliche, organische Verbindung, ausgewählt aus Sulfoxiden, N,N'-Dialkylamiden, Ketonen, Nitrilen, und zyklischen Ethern.

2. Reagenz-Zusammensetzung gemäß Anspruch 1, wobei das wasserlösliche Polymer mit einem Gewichtsmittel der Molmasse von 8.000 oder mehr eines oder mehrere ausgewählt aus der Gruppe bestehend aus Polyalkylenglykolen, Zellulosen, Vinyl-basierenden Polymeren, Amid-basierenden Polymeren, und einem Polyanion ist.

3. Reagenz-Zusammensetzung gemäß Anspruch 2, wobei das Polyanion ein Polysaccharidanion ist.

4. Reagenz-Zusammensetzung gemäß Anspruch 3, wobei das Polysaccharidanion eines oder mehrere ausgewählt aus der Gruppe bestehend aus Dextransulfat, Heparansulfat, Chondroitinsulfat, Dermatansulfat, Keratansulfat, Hyaluronsäure, Heparin, und deren Salzen ist.

5. Reagenz-Zusammensetzung gemäß einem der Ansprüche 1 bis 4, wobei die aprotische, wasserlösliche, organische Verbindung eine oder mehrere ausgewählt aus der Gruppe bestehend aus Sulfoxiden und N,N'-Dialkylamiden ist.

6. Reagenz-Zusammensetzung gemäß einem der Ansprüche 1 bis 5, wobei das Salz eines zweiwertigen oder dreiwertigen Metalls eines oder mehrere ausgewählt aus der Gruppe bestehend aus einem Magnesiumsalz, einem Calciumsalz, und einem Aluminiumsalz ist.

7. Reagenz-Zusammensetzung gemäß einem der Ansprüche 1 bis 6, wobei das nicht-ionische Tensid eines oder mehrere ausgewählt aus der Gruppe bestehend aus Polyoxyethylen-Sorbitanmonolaurat, Polyoxyethylen-Sorbitanmonostearat, Polyoxyethylen-Sorbitantristearat, und Polyoxyethylen-Sorbitanmonooleat ist.

8. Verwendung der Reagenz-Zusammensetzung gemäß einem der Ansprüche 1 bis 7 in der Nukleinsäure-Chromatographie oder Immunochromatographie, wobei kolloidale Goldpartikel als Markierungsstoff verwendet werden.

9. Verwendung der Reagenz-Zusammensetzung gemäß einem der Ansprüche 1 bis 7 für die Nukleinsäure-Chromatographie.

10. Verwendung der Reagenz-Zusammensetzung gemäß einem der Ansprüche 1 bis 7 für die Immunochromatographie.

11. Verwendung der Reagenz-Zusammensetzung gemäß einem der Ansprüche 1 bis 7 als einen Entwickler bei der Nukleinsäure-Chromatographie oder Immunochromatographie.

12. Messverfahren mittels der Nukleinsäure-Chromatographie oder Immunochromatographie, umfassend das Entwickeln einer zu messenden Probe unter Verwendung der Reagenz-Zusammensetzung gemäß einem der Ansprüche 1 bis 7.

13. Kit zur Messung mittels der Nukleinsäure-Chromatographie oder Immunochromatographie, umfassend die Reagenz-Zusammensetzung gemäß einem der Ansprüche 1 bis 7 als einen Entwickler einer zu messenden Probe.

## Revendications

1. Composition de réactifs pour chromatographie ou immunochromatographie d'acide nucléique, comprenant :
un polymère soluble dans l'eau ayant une masse moléculaire moyenne en masse de 8000 ou plus,
un sel d'un métal divalent ou trivalent,
un tensioactif non ionique,
et un composé organique aprotique soluble dans l'eau qui est choisi parmi les sulfoxydes, les N,N'-dialkylamides, les cétones, les nitriles et les éthers cycliques.

2. Composition de réactifs selon la revendication 1, dans laquelle le polymère soluble dans l'eau ayant une masse moléculaire moyenne en masse de 8000 ou plus est d'une ou plusieurs sortes choisies dans le groupe constitué des poly(alkylène glycols), des celluloses, des polymères à base de vinyle, des polymères à base d'amide et d'un polyanion.

3. Composition de réactifs selon la revendication 2, dans laquelle le polyanion est un anion de polysaccharide.

4. Composition de réactifs selon la revendication 3, dans laquelle l'anion de polysaccharide est d'une ou plusieurs sortes choisies dans le groupe constitué du sulfate de dextrane, du sulfate d'héparane, du sulfate de chondroïtine, du sulfate de dermatane, du sulfate de kératane, de l'acide hyaluronique, de l'héparine et de leurs sels.

5. Composition de réactifs selon l'une quelconque des revendications 1 à 4, dans laquelle le composé organique aprotique soluble dans l'eau est d'une ou plusieurs sortes choisies dans le groupe constitué des sulfoxydes et des N,N'-dialkylamides.

6. Composition de réactifs selon l'une quelconque des revendications 1 à 5, dans laquelle le sel d'un métal divalent ou trivalent est d'une ou plusieurs sortes choisies dans le groupe constitué d'un sel de magnésium, d'un sel de calcium et d'un sel d'aluminium.

7. Composition de réactifs selon l'une quelconque des revendications 1 à 6, dans laquelle le tensioactif non ionique est d'une ou plusieurs sortes choisies dans le groupe constitué du monolaurate de sorbitan polyoxyéthyléné, du monostéarate de sorbitan polyoxyéthyléné, du tristéarate de sorbitan polyoxyéthyléné et du monooléate de sorbitan polyoxyéthyléné.

8. Utilisation de la composition de réactifs selon l'une quelconque des revendications 1 à 7 pour chromatographie ou immunochromatographie d'acide nucléique, dans laquelle des particules d'or colloïdal sont utilisées comme substance de marquage.

9. Utilisation de la composition de réactifs selon l'une quelconque des revendications 1 à 7 pour chromatographie d'acide nucléique.

10. Utilisation de la composition de réactifs selon l'une quelconque des revendications 1 à 7 pour immunochromatographie.

11. Utilisation de la composition de réactifs selon l'une quelconque des revendications 1 à 7 comme révélateur en chromatographie ou immunochromatographie d'acide nucléique.

12. Procédé de mesure par chromatographie ou immunochromatographie d'acide nucléique, comprenant le développement d'un spécimen à mesurer en utilisant la composition de réactifs selon l'une quelconque des revendications 1 à 7.

13. Kit de mesure par chromatographie ou immunochromatographie d'acide nucléique comprenant la composition de réactifs selon l'une quelconque des revendications 1 à 7 comme révélateur d'un spécimen à mesurer.
